# EUROPÄISCHE PATENTSCHRIFT

(11) **EP 4 262 612 B1**
(45) Veröffentlichungstag und Bekanntmachung des Hinweises auf die Patenterteilung: **21.05.2025**
(21) Anmeldenummer: 23706767.3
(22) Anmeldetag: 22.02.2023
(51) Int. Cl.: A61C 1/14, A61B 17/16

(54) **INSTRUMENTENKOPF MIT HEBELMECHANISMUS ZUM SPANNEN EINES BEHANDLUNGSWERKZEUGS**
INSTRUMENT HEAD WITH LEVER MECHANISM FOR CLAMPING A TREATMENT TOOL
TÊTE D'INSTRUMENT AVEC MÉCANISME DE LEVIER POUR SERRAGE D'UN OUTIL DE TRAITEMENT

(30) Priorität: 10.03.2022 DE 102022105626
(43) Veröffentlichungstag der Anmeldung: 25.10.2023
(73) Patentinhaber: KaVo Dental GmbH, 88400 Biberach (DE)
(72) Erfinder: BUTSCHER, Jürgen, 88299 Leutkirch (DE); RÖSCH, Thomas, 88447 Warthausen (DE)
(74) Vertreter: Thun, Clemens
(86) Internationale Anmeldenummer: PCT/EP2023/054416
(87) Internationale Veröffentlichungsnummer: WO 2023/169822

(56) Entgegenhaltungen:
- US-A- 5 040 980
- US-A- 5 090 906

## Beschreibung

Die Erfindung betrifft ein medizinisches, insbesondere dentalmedizinisches, Handinstrument mit einem Instrumentenkopf und einem im Instrumentenkopf aufgenommenen medizinischen Behandlungswerkzeug, wobei das Behandlungswerkzeug über einen Haltemechanismus mit dem Instrumentenkopf lösbar gekoppelt ist.

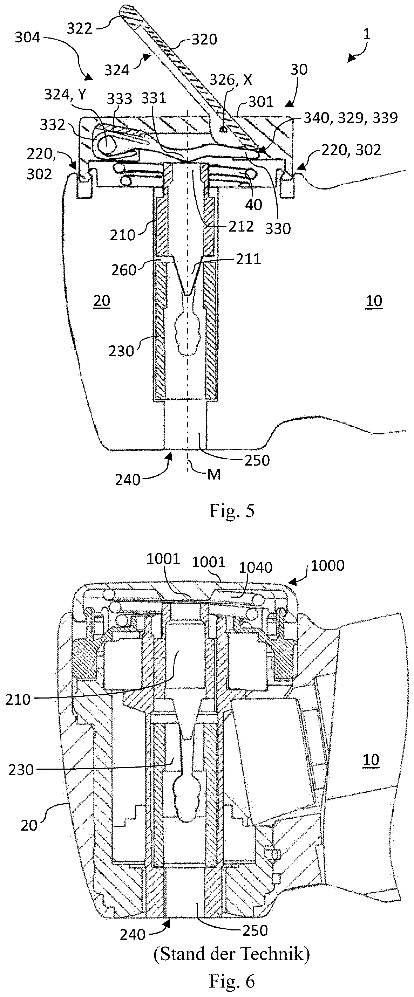

Derartige Handinstrumente bestehen üblicherweise im Wesentlichen aus einer Griffhülse und einem Instrumentenkopf, auch als Kopfstück bezeichnet, welches zur einfachen und präzisen Bedienung des Handinstruments an einem vorderen Ende der Griffhülse angeordnet ist. In Figur 6 ist ein solches übliches Kopfstück 20, welches an einem Ende einer Griffhülse 10 eines medizinischen Handinstruments 1 angeordnet ist, gezeigt. Das Kopfstück 20 weist hierbei eine Werkzeugaufnahmeöffnung 240 auf, sodass anwendungsspezifisch unterschiedliche medizinische bzw. dentalmedizinische Behandlungswerkzeuge 7 in das Kopfstück 20 eingesetzt und mit dem Handinstrument 1 gekoppelt werden können. Hierzu ist im Inneren des Kopfstücks 20 ein Haltemittel 230 zur drehbaren Halterung des jeweiligen Behandlungswerkzeugs 7, beispielsweise eines zahnärztlichen Bohrers, vorhanden. Mit den Haltemitteln 230 ist weiterhin auch ein Löseelement 210 gekoppelt, welche bei entsprechender Betätigung die Halterung des Behandlungswerkzeugs 7 im Haltemittel 230 löst. Während des Betriebs rotieren das Behandlungswerkzeug 7 und die mit dem Behandlungswerkzeug 7 gekoppelten Bauteile wie Löseelement 210 und Haltemittel 230 mit bis zu 450.000 Umdrehungen pro Minute.

Bislang wird zur Halterung des Behandlungswerkzeugs 7 üblicherweise eine Spannzange 230 verwendet, welche mittels des keilförmigen Löseelements 210 durch Drücken eines Druckknopfs 1000, betätigt wird, sodass die Halterung des Behandlungswerkzeugs 7 gelöst wird. Der Druckknopf 1000 weist hierfür üblicherweise ein Druckelement 1001 und eine Druckfeder 1040 auf, welche den Druckknopf 1000 entgegen der Betätigungsrichtung des Druckknopfs 1000 wegdrückt. Beim Betätigen des Druckknopfes 1000 wird die Druckfeder 1040 zusammengestaucht, wobei dann das Druckelement 1001 das Löseelement 210 kontaktiert und dieses weiter in das Haltemittel 230 hineinverschiebt. Bei genügend großer Betätigung des Druckknopfes 1000 und somit entsprechend großer Verschiebung des Löseelements 210 wird die Halterung des Behandlungswerkzeugs 7 im Haltemittel 230 gelöst, und das Behandlungswerkzeug 7 kann aus dem Werkzeugkanal 240 des Kopfstücks 20 entnommen werden. Solche Druckknopfmechanismen sind üblicherweise in einem auf das Kopfteil 20 des Handinstruments 1 aufschraubbaren Deckel implementiert.

Diese bisherige Anordnung mit dem Druckknopf 1000 und der Druckfeder 1040 zur Lösung des Haltemechanismus bedarf allerdings einen entsprechend hohen Kraftaufwand, da neben der zu überwindenden Federkraft der Druckfeder 1040 auch die Interaktion zwischen Haltemittel 230 und Löseelement 210 einer gewissen Kraft - üblicherweise circa 50 Newton - erfordert. Somit ist dieser bisherige Mechanismus schwer bedienbar und unhandlich.

Weiterhin resultiert mit diesem bekannten Mechanismus bereits ein geringfügiges Betätigen des Druckknopfes 1000 zur Kontaktierung des Druckelements 1001 mit dem Löseelement 210, wobei hierfür lediglich die Federkraft der Druckfeder 1040 zu überwinden ist. Ein Kontaktieren des Druckknopfes 1000 mit dem Löseelement 210 stellt allerdings während des Betriebs eine große Gefahrenquelle dar, da bei Kontakt des Druckelements 1001 mit dem mit hoher Drehzahl drehenden Löseelement 210 eine erhebliche Reibung entsteht, wodurch der Druckknopf 1000 äußerst schnell äußerst heiß wird, was wiederum eine große Verletzungsgefahr darstellt. Ein solches geringfügiges Eindrücken kann bereits durch einen ungewollten Kontakt des Druckknopfes 1000 im Mund eines Patienten mit einem Zahn oder einer Wange geschehen, sodass hier eine Verbrennungsgefahr für den Patienten besteht. Ebenfalls besteht eine Verbrennungsgefahr für den behandelnden Arzt, da durchaus denkbar ist, dass dieser versehentlich selbst während der Behandlung den Druckknopf 1000 geringfügig eindrückt.

Solche bekannten Instrumentenköpfe mit einem Druckknopfmechanismus zur Lösung der Halterung eines Behandlungswerkzeugs sind daher schwer und unhandlich zu bedienen und bergen darüber hinaus auch ein Gefahrenpotential sowohl für den behandelnden Arzt als auch den Patienten.

Ferner wird in der EP 1 627 611 A1 ein Handstückkopf für ein medizinisches Handstück beschrieben, bei dem eine Hubbewegung entlang einer Achse auf ein im Handstückkopf angeordnetes Werkzeug übertragen wird. Hierbei ist das Werkzeug bezüglich einer Rotation um die Achse in jeder beliebigen Position fixierbar, wobei abhängig vom Bedarf das Werkzeug um die eigene Achse rotieren kann oder durch einen Feststellmechanismus von einer solchen Rotation abgehalten wird.

Für den Feststellmechanismus ist vorgesehen eine Klemmverbindung herzustellen. Hierfür weist der Handstückkopf eine Hülse zur Aufnahme des Werkzeugs auf, an welcher ein erstes Klemmteil angeordnet ist. Zudem ist als Teil eines Vorsatzes an einem Griffteil des Handstücks ein zweites Klemmteil vorgesehen, welches in Richtung des Handstückkopfs geschoben werden kann und dabei derart mit dem ersten Klemmteil interagiert, dass die angestrebte Klemmverbindung entsteht. Der hier beschriebene Klemmmechanismus dient also in erster Linie einer Arretierung der das Werkzeug haltenden Hülse in Bezug auf die Rotationsachse, nicht jedoch einer lösbaren Halterung des Werkzeugs.

Die US 5,090,906 A beschreibt ein zahnärztliches Handstück in dessen Kopf ein Schneidschaft eines Instruments aufgenommen und mit einer Druckknopf-Steuereinrichtung festgeklemmt wird. Die Druckknopf-Steuereinrichtung ist hierbei gegen eine Spannfeder verschiebbar. Die Steuervorrichtung besteht im allgemeinen aus mindestens einem Umkehrelement zur Aufnahme von Drücken, die auf Bewegungen des Druckknopfes ansprechen, und zur Umwandlung dieser Bewegungen in eine Zugbewegung, die durch das Umkehrelement auf den Stößel ausgeübt wird, der zur Steuerung der Spannzange dient, die den Schaft des Instruments aufnimmt.

Die US 5,040,980 A zeigt ein zahnärztliches Handstück mit einem Bohreraufnahmefutter mit federnden Klemmbacken, die eine Klemmkraft auf den Schaft eines Bohrers ausüben, um den Bohrer funktionsfähig am Handstückrotor zu befestigen, wobei ein solches Handstück auch einen hebelbetätigten Bohrerfreigabemechanismus aufweist, um die Klemmbacken auseinander zu drücken, wenn ein Bohrer entfernt und ersetzt werden soll.

Der Erfindung liegt die Aufgabe zugrunde, ein medizinisches Handinstrument mit einem verbesserten Mechanismus zur Bedienung des Löseelements und des Haltemittels zur Lösung der Halterung eines in das Handinstrument eingesetzten Behandlungswerkzeugs, und einen Deckel mit einem solchen verbesserten Mechanismus für ein medizinisches Handinstrument anzugeben.

Diese Aufgabe wird gemäß der Erfindung mit dem in den unabhängigen Ansprüchen genannten Gegenstand gelöst. Besondere Ausführungsarten der Erfindung sind in den abhängigen Ansprüchen angegeben.

Gemäß der Erfindung ist ein medizinisches, insbesondere dentalmedizinisches Handinstrument mit einer Griffhülse und einem Kopfstück vorgesehen, welches an einem vorderen Ende der Griffhülse angeordnet ist und eine Werkzeugaufnahmeöffnung aufweist, wobei im Inneren des Kopfstücks ein Haltemittel zur drehbaren Halterung eines Behandlungswerkzeugs (beispielsweise eines zahnärztlichen Bohrers) angeordnet ist. Weiterhin ist vorgesehen, dass das Haltemittel entlang einer Drehachse für das Behandlungswerkzeug angeordnet ist, und dass im Inneren des Kopfstücks entlang dieser Drehachse ein Löseelement angeordnet ist, welches dazu ausgebildet ist, mit dem Haltemittel zu interagieren, wobei das Löseelement bei einer Interaktion mit dem Haltemittel die Halterung des Behandlungswerkzeugs löst. Erfindungsgemäß weist das Kopfstück weiterhin einen Deckel auf, wobei der Deckel einen Hebelmechanismus umfasst, der einen ersten Hebel und einen zweiten Hebel aufweist, welche zusammenwirken um die Halterung des Behandlungswerkzeugs zu lösen.

Dadurch wird der Mechanismus zur Lösung der Halterung des Behandlungswerkzeugs mittels eines Hebelmechanismus realisiert, wobei insbesondere durch die Gestaltung zweier zusammenwirkender Hebel die aufzuwendende Kraft zur Lösung der Halterung stark minimiert wird.

Neben dem erfindungsgemäßen Handinstrument ist weiterhin erfindungsgemäß ein Deckel für ein Kopfstück eines medizinischen, insbesondere dentalmedizinische Handinstruments vorgesehen.

Dadurch ist der Deckel insbesondere auch auf älteren und bereits bekannten medizinischen Handinstrumenten anbringbar, derart, dass der damit verbundene verbesserte Mechanismus zur Lösung des Haltemechanismus auch mit anderen und insbesondere älteren Handinstrumenten verwendbar ist. Damit sind also die Vorteile der größeren Benutzungssicherheit und einfacheren Bedienbarkeit des erfindungsgemäßen Deckels auch für Benutzer älterer Handinstrumente erfahrbar.

Die nachfolgenden Ausführungen und Gestaltungen des Deckels sind daher gleichermaßen für den erfindungsgemäßen Deckel wie auch für das erfindungsgemäße Handinstrument gültig.

Erfindungsgemäß wirkt hierbei der erste Hebel bei Betätigung auf ein freies Ende des zweiten Hebels. Dadurch wird eine besonders effiziente Hebelwirkung durch Zusammenwirken des ersten und des zweiten Hebels erzielt, sodass sich die Erleichterung der benötigten Krafteinwirkung zur Lösung der Halterung mittels Löseelement und Haltemittel potenziert.

Vorzugsweise sind eine Schwenkachse des ersten Hebels und eine Schwenkachse des zweiten Hebels parallel zueinander angeordnet und stehen senkrecht auf einer Ebene, welche entlang der Drehachse verläuft. Mit einer derartigen Gestaltung lässt sich eine besonders platzsparende Anordnung und eine besonders vorteilhafte Kraftübertragung vom ersten Hebel auf den zweiten Hebel erzielen.

Vorzugsweise sind die Schwenkachse des ersten Hebels und die Schwenkachse des zweiten Hebels in Bezug auf die Drehachse einander gegenüberliegend an dem Deckel angeordnet. Hierdurch lässt sich eine besonders vorteilhafte Anordnung der beiden Hebel realisieren. Weiterhin bevorzugt sind in Richtung der Drehachse beide Schwenkachsen zueinander versetzt. Mit der zusätzlichen versetzten Anordnung, lässt sich eine entsprechend vergrößerte Hebelwirkung realisieren, da die beiden Schwenkachsen entsprechen voneinander beabstandet positioniert sind, und so die potentielle Hebellänge vergrößert ist.

Vorzugsweise weist der zweite Hebel eine Durchbiegung auf, welche zur Interaktion mit dem Löseelement auf dieses drückt, wobei durch die Durchbiegung eine auf das Löseelement wirkende Kraft begrenzt wird. Hierbei ist insbesondere vorgesehen, dass der zweite Hebel durch die Durchbiegung eine Elastizität hat, sodass ab einer bestimmten vorliegenden Hebelkraft, die auf die Löseeinheit wirkende Kraft begrenzt wird und eine zu große Krafteinwirkung verhindert wird. Hierdurch wird die Benutzungssicherheit des Handinstruments erhöht, da eine Beschädigung von Bauteilen des Handinstruments vermieden werden.

Vorzugsweise ist der zweite Hebel im Bereich seiner Schwenkachse mit einer Rückstellfeder, insbesondere einer Schenkelfeder, versehen, welche dazu ausgebildet ist, bei ausbleibender Betätigung des ersten Hebels den ersten und zweiten Hebel in eine Ruheposition zu überführen, wobei in der Ruheposition der zweite Hebel von dem Löseelement beabstandet ist. Dadurch wird in einfacher Weise bewerkstelligt, dass nach Betätigen des Hebelmechanismus - mit erstem und zweitem Hebel - beide Hebel selbstständig in ihre Ruheposition zurückgelangen, sodass insbesondere eine Kontaktierung des zweiten Hebels mit dem Löseelement verhindert ist. Somit wird durch diese Implementierung die Benutzungssicherheit weiter verbessert.

Vorzugsweise ist der erste Hebel an seinem der Schwenkachse zugewandten Endbereich mit seitlichen Vorwölbungen versehen, welche der Lagerung des ersten Hebels in dem Deckel dienen und den Endbereich des zweiten Hebels jeweils seitlich übergreifen. Durch diese Beschaffenheit des ersten Hebels wird der zweite Hebel bei Nichtverwendung des Hebelmechanismus (Ruheposition) gesichert gelagert, wobei weiterhin auch die Position des zweiten Hebels zum ersten Hebel festgesetzt wird. Diese seitlichen Vorwölbungen des ersten Hebels erhöhen weiterhin auch die Stabilität des ersten Hebels.

Vorzugsweise ist der erste Hebel an seinem von der Schwenkachse entfernten Endbereich mit einem Griffelement versehen. Hierdurch wird die Bedienbarkeit des Hebelmechanismus zur Lösung des Haltemechanismus vereinfacht, wobei weiterhin auch die Kraftübertragung auf den ersten Hebel verbessert ist. Auch stabilisiert das Griffelement den ersten Hebel zusätzlich.

Vorzugsweise ist der erste Hebel an seiner dem zweiten Hebel zugewandten Unterseite mit einer Ausnehmung versehen, welche in einer Ruheposition der beiden Hebel zumindest einen Teil des zweiten Hebels aufnimmt. Hierdurch wird ein Verrutschen des zweiten Hebels verhindert, wobei zudem der Deckel mit einer geringeren Bauhöhe realisierbar ist, da die beiden Hebel platzsparend angeordnet sind.

Vorzugsweise ist der Deckel reversibel mit dem Kopfstück des Handinstruments koppelbar, wobei hierfür bevorzugt der Deckel ein Gewinde und das Kopfstück ein entsprechendes Gegengewinde aufweist. Hierdurch lassen sich besonders einfach und vorteilhaft Wartungs-, Reinigungs- bzw. Reparaturarbeiten im Inneren des Kopfstücks wie auch am Deckel durchführen, wobei weiterhin die einzelnen Bauteile des Deckels wie auch des Kopfstücks so besonders einfach zugänglich sind.

Vorzugsweise weist der Deckel eine Feststellvorrichtung auf, wobei der Hebelmechanismus mittels der Feststellvorrichtung wahlweise in einer gewünschten Orientierung bezüglich der Drehachse arretierbar ist. Hierdurch lässt sich die Betätigungsrichtung des Hebelmechanismus in eine vom Benutzer präferierte Orientierung bringen, sodass der Hebelmechanismus besonders einfach und handlich individualisierbar ist.

In einer speziellen vorzugsweisen Ausführung ist der Hebelmechanismus entlang der Drehachse beweglich gelagert und der Hebelmechanismus ist mittels Federkraft in eine von dem Löseelement beabstandete Position vorgespannt, wobei der Hebelmechanismus entgegen der Federkraft herabdrückbar ist, um in einer Ruheposition der beiden Hebel im herabgedrückten Zustand das Löseelement zu kontaktieren. Mit dieser speziellen Ausgestaltung, ist neben dem Hebelmechanismus mit erstem und zweitem Hebel ein zusätzlicher Druckmechanismus zur Lösung des Haltemechanismus implementiert, wobei hierfür der Deckel mitsamt der in der Ruheposition verweilenden Hebel herunterdrückbar ist, derart, dass der zweite Hebel mit dem Löseelement kontaktiert wird, wodurch wiederum die Halterung im Haltemittel gelöst wird. Insofern bietet diese Ausführungsform zwei Mechanismen zur Lösung des Haltemechanismus.

Vorzugsweise ist das Haltemittel eine Spannzange, und/oder das Löseelement ein Keilelement mit einem keilförmigen Kopfbereich und einem planen Bodenbereich. Hierdurch lässt sich ein besonders einfacher aber dennoch robuster Haltemechanismus aus Löseelement und Haltemittel realisieren.

Weiterhin vorzugsweise ist der erste Hebel als zweiarmiger Hebel und der zweite Hebel als einarmiger Hebel ausgebildet. Hierdurch wird zum einen die Hebelwirkung des Hebelmechanismus verstärkt, wobei zugleich die beiden Hebel in platzsparender Weise im Deckel positionierbar sind.

Die Erfindung wird im Folgenden anhand eines Ausführungsbeispiels und mit Bezug auf die Zeichnungen näher erläutert. Es zeigen:
- Fig. 1: eine schematische Seitendarstellung einer Ausführungsform eines erfindungsgemäßen medizinischen Handinstruments mit einem in das Kopfstück des Handinstruments eingesetzten Behandlungswerkzeug;
- Fig. 2: eine schematische Querschnittsdarstellung einer beispielhaften ersten Ausführungsform eines erfindungsgemäßen Deckels und medizinischen Handinstruments;
- Fig. 3A: eine schematische Querschnittsdarstellung einer beispielhaften zweiten Ausführungsform eines erfindungsgemäßen Deckels für ein medizinisches Handinstruments in einer Bedienposition des Hebelmechanismus;
- Fig. 3B: eine perspektivische Darstellung der Ausführungsform aus Figur 3A eines erfindungsgemäßen Deckels für ein medizinisches Handinstrument in einer Bedienposition des Hebelmechanismus;
- Fig. 4A: eine schematische Querschnittsdarstellung der Ausführungsform aus Figur 3A eines erfindungsgemäßen Deckels für ein medizinisches Handinstruments in der Ruheposition des Hebelmechanismus;
- Fig. 4B: eine perspektivische Darstellung der Ausführungsform aus Figur 3A eines erfindungsgemäßen Deckels für ein medizinisches Handinstruments in der Ruheposition des Hebelmechanismus;
- Fig. 5: eine schematische Querschnittsdarstellung einer beispielhaften dritten Ausführungsform eines erfindungsgemäßen Deckels und medizinischen Handinstruments;
- Fig. 6: eine schematische Querschnittsdarstellung des Kopfbereichs eines aus dem Stand der Technik bekannten medizinischen Handinstruments.

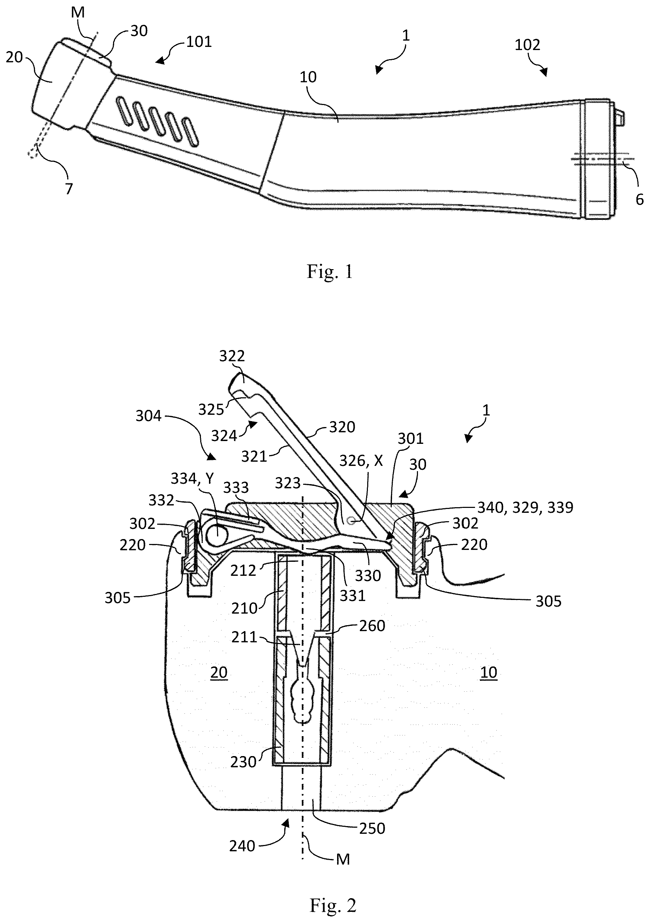

Die Figur 1 zeigt den prinzipiellen Aufbau eines erfindungsgemäßen medizinischen, und insbesondere dentalmedizinischen, Handinstruments 1, welches an seinem vorderen Ende 101 ein Kopfstück 20 aufweist, wobei das Kopfstück 20 wiederum mit der Griffhülse 10 des Handinstruments 1 gekoppelt ist. Am hinteren Ende 102 des Handinstruments 1 ist üblicherweise ein Anschluss für eine Zuleitung 6 vorgesehen, über welchen das Handinstrument 1 beispielsweise mit einer Energieversorgungsquelle gekoppelt werden kann, insbesondere kann vorgesehen sein hierüber das Handinstrument 1 mit elektrischer Energie oder mechanischer Energie (beispielsweise Rotationsenergie) zu versorgen.

Der für die Erfindung wesentliche Teil des Handinstruments ist in den Figuren 2 bis 5 näher dargestellt, wobei hier der Bereich des Kopfstücks 20 detaillierter dargestellt ist. Die Erfindung zielt auf einen verbesserten Mechanismus zur Lösung des Haltemechanismus für ein Behandlungswerkzeug 7 im Kopfstück 20 des Handinstruments 1 ab. Dieser Mechanismus ist hierbei in einem Deckel 30 des Handinstruments 1 eingebaut, und ist als Hebelmechanismus 304 ausgeführt. Der Deckel 30 ist in den verschiedenen hier gezeigten beispielhaften Ausführungen stets mit dem Kopfstück 20 reversibel koppelbar.

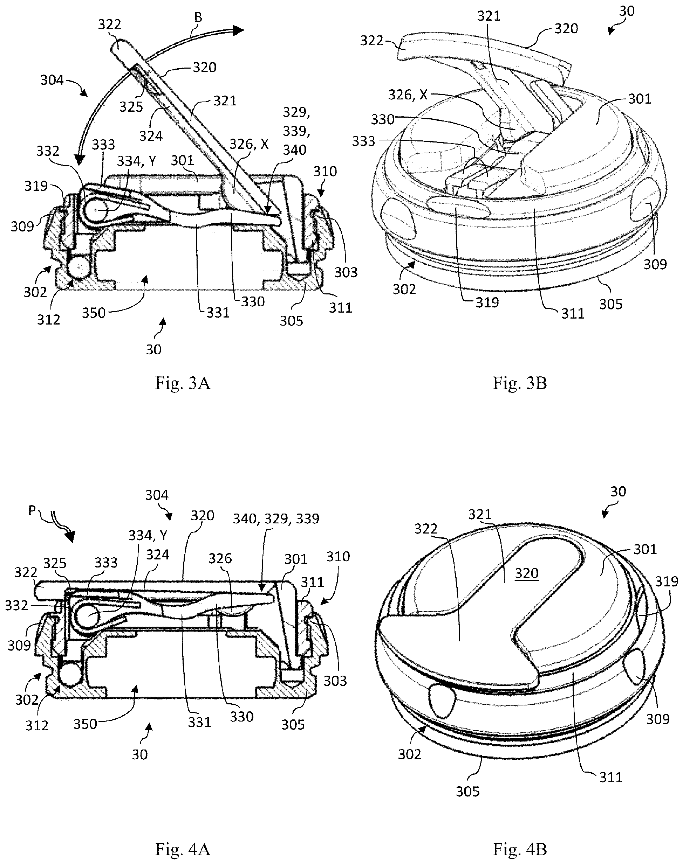

Der Hebelmechanismus 304 weist einen ersten Hebel 320 und einen zweiten Hebel 330 auf, welche miteinander zusammenwirken um die Halterung des Behandlungswerkzeugs 7 zu lösen. Hierzu interagiert der Hebelmechanismus 304 mit dem Löseelement 210, sodass das Löseelement 210 wiederum das Haltemittel 230 betätigt (beziehungsweise öffnet) und ein entsprechendes Behandlungswerkzeug 7 in das Kopfstück 20 des Handinstruments 1 eingesetzt oder herausgenommen werden kann. Durch den Hebelmechanismus 304 wird die vom Benutzer aufzubringende Kraft minimiert. Weiterhin erfolgt die Betätigung B des ersten Hebels 320 in entgegengesetzter Richtung zur Interaktion des zweiten Hebels 330 mit dem Löseelement 210, sodass durch diese erforderliche vom Deckel 30 abspreizende Betätigungsbewegung B des ersten Hebels 320 Hebelbewegung, wie sie exemplarisch in der Figur 3A illustriert ist, ein unbeabsichtigtes Auslösen des Hebelmechanismus 304 zur Lösung des Haltemechanismus verhindert. Hierdurch besteht für den Benutzer wie auch für einen Patienten keine Verbrennungsgefahr durch ein unbeabsichtigtes Betätigen, da eine Kraftausübung auf den Deckel 30 und insbesondere den Hebel 320 - beispielsweise durch einen Kontakt mit einer Wange oder einem Zahn eines Patienten, oder einem Finger des behandelnden Arztes - den Hebelmechanismus 304 nicht auslöst, und so während des Betriebs des Handinstruments 1 ein Kontaktieren des Hebelmechanismus 304 mit schnell rotierenden Bauteilen vermieden wird.

Unabhängig von der konkreten Ausführung des Deckels 30, ist der darin implementierte Hebelmechanismus 304 stets dazu ausgebildet bei Betätigung B mit dem Löseelement 210 zu interagieren, sodass dieses wiederum das Haltemittel 230 löst. Das Haltemittel 230 und das Löseelement 230 bilden hierbei einen sogenannten Haltemechanismus. Wie auch in der Figur 2 dargestellt ist das Haltemittel 230 bevorzugt eine Spannzange - also eine röhrenförmige Hülse, welche eine Stufenbohrung und mindestens einen Schlitz aufweist. Das Haltemittel 230 wird bei Betätigung, mittels des Löseelements 210, welches bevorzugt einen keilförmigen Kopfbereich 211 und einen planen Bodenbereich 212 aufweist, aufgespreizt, wobei hierfür beispielsweise der keilförmige Kopfbereich 211 des Löseelements 210 mit dem Schlitz der Spannzange interagiert, und so die Hülse der Spannzange dehnt, sodass das Behandlungswerkzeug 7 über die Werkzeugaufnahmeöffnung 240 und den Werkzeugkanal 250 des Kopfstücks 20 in das Haltemittel 230 eingeführt oder herausgenommen werden kann. Das Löseelement 210 muss hierfür aktiv in Richtung des Haltemittels 230 gedrückt werden - was insbesondere durch Betätigung B des Hebelmechanismus 304 erzielt wird -, da die Interaktion der beiden Komponenten des Haltemechanismus selbsthemmend ist, also das Löseelement 210 durch das Haltemittel 230 selbst herausgedrückt wird.

Innerhalb des Kopfteils 20 sind das Löseelement 210 und das Haltemittel 230 in einer dafür vorgesehenen Ausnehmung 260 angeordnet. Der Durchmesser dieser Ausnehmung 260 ist hierbei größer als der Durchmesser der Werkzeugaufnahmeöffnung 240, respektive des Werkzeugkanals 250, sodass die Komponenten des Haltemechanismus sicher im Inneren des Kopfstücks 20 gelagert sind. Insbesondere ist vorgesehen, dass sowohl das Haltemittel 230 als auch das Löseelement 210 zusammen mit dem Behandlungswerkzeug 7 rotierbar gelagert sind, da diese sich bei Benutzung des Handinstruments 1 mit der Drehzahl des Behandlungswerkzeugs 7 mitdrehen. Hierbei übernehmen die Komponenten des Haltemechanismus zusätzlich eine werkzeugzentrierende Aufgabe.

In Figur 2 ist eine erste beispielhafte Ausführungsform des Deckels 30 gezeigt. Der Deckel 30 ist hierbei über ein Gewinde 302 mit dem Kopfstück 20, welches ein entsprechendes Gegengewinde 220 aufweist, verschraubt. Der Hebelmechanismus 304 ist im Mittelstück 301 des Deckels 30 platziert, wobei in dieser ersten beispielhaften Ausführungsform das Mittelstück 301 frei drehbar relativ zum Mittelstück 301 ringartig umgebenen Außenstück 305 angeordnet ist, sodass situationsbedingt die Ausrichtung des Hebelmechanismus 304 und insbesondere des vom Benutzer bedienten ersten Hebels 320 verändert werden kann. Auf dem Außenstück 305 ist das Gewinde 302 angeordnet.

Der erste Hebel 320 (auch als oberer Hebel 320 bezeichnet) weist ein Griffelement 322 auf, welches die Bedienung des Hebelmechanismus 304 erheblich vereinfacht. Auf der dem Griffelelement 322 gegenüberliegenden Seite ist der erste Hebel 320 mit seitlichen Vorwölbungen 323 versehen, welche jeweils eine Aufhängung 326 aufweisen, welche der Lagerung des Hebels 320 in dem Deckel 30 dienen und den Endbereich des zweiten Hebels 330 jeweils seitlich übergreifen. Dadurch sind die zwei Hebel 320, 330 in platzsparender Weise in der Ruheposition P, wie in der Figur 4A angedeutet, im Deckel 30 angeordnet. Die seitlichen Vorwölbungen 323 bilden mit ihren Aufhängungen 326 die Schwenkachse X des ersten Hebels 320, welche so am ersten Hebel 320 angeordnet ist, dass dieser ein zweiarmiger Hebel ist. Die Schwenkachse X des ersten Hebels 320 ist insofern sowohl vom Griffelement 322 als auch vom dem Griffelement 322 gegenüberliegenden Ende des ersten Hebels 320 beabstandet.

Das dem Griffelement 322 gegenüberliegenden Ende des ersten Hebels 320 bildet die Kontaktfläche 329 des ersten Hebels 320, welche bei Betätigung B des Hebelmechanismus 304 auf ein freies Ende des zweiten Hebel 330 wirkt. Dieses freie Ende des zweiten Hebels 330 weist die Kontaktfläche 339 auf, welche mit der Kontaktfläche 329 des ersten Hebels 320 interagiert. Das Griffelement 322 und die Kontaktfläche 329 des ersten Hebels 320 sind über einen länglichen Steg 321 verbunden. Die Kontaktierung 340 zwischen erstem Hebel 320 und zweiten Hebel 330 erfolgt über die Kontaktflächen 329, 339 ist stets vorhanden und auch in der Ruheposition P der beiden Hebel 320, 330 nicht unterbrochen, sodass bereits ein geringfügiges Betätigen B des ersten Hebels 320 in eine Bewegung des zweiten Hebels 330 resultiert.

Weiterhin ist der erste Hebel 320 an seiner dem zweiten Hebel 330 zugewandten Unterseite mit einer Ausnehmung 324 versehen, welche in einer Ruheposition P der beiden Hebel 320, 330 zumindest einen Teil des zweiten Hebels 330 aufnimmt, sodass der zweite Hebel 330 platzsparend darin eingebettet werden kann.

Der zweite Hebel 330 (auch als unterer Hebel 330 bezeichnet) ist als einarmiger Hebel ausgeführt, wobei dessen Aufhängung 334 am der Kontaktfläche 339 des zweiten Hebels 330 gegenüberliegenden Ende des zweiten Hebels 330 angeordnet ist. Diese Aufhängung 334 des zweiten Hebels 330 bildet zudem dessen Schwenkachse Y.

Zwischen der Kontaktfläche 339 und der Aufhängung 334 weist der zweite Hebel eine Durchbiegung 331 auf, welche zur Interaktion mit dem Löseelement 210 auf dieses drückt, wobei durch die Durchbiegung 331 eine auf das Löseelement 210 wirkende Kraft begrenzt wird. Durch diese Durchbiegung 331 hat der zweite Hebel 330 eine Elastizität, sodass ab einer bestimmten Hebelkraft, sich der untere Hebel 330 nicht weiter durchbiegt und somit eine zu große Krafteinwirkung auf das Löseelement 210 verhindert wird. Die Durchbiegung 331 ist hierbei zentriert im Deckel 30 angeordnet, sodass sie unabhängig von der Ausrichtung des Mittelstücks 301 stets auf der Drehachse M platziert ist, und somit bei Betätigung B mit dem Löseelement 210 interagiert und eine optimale Kraftübertragung sicherstellt.

Der zweite Hebel 330 ist weiterhin im Bereich seiner Schwenkachse Y mit einer Rückstellfeder 332, insbesondere einer Schenkelfeder, versehen, welche dazu ausgebildet ist, bei ausbleibender Betätigung B des ersten Hebels 320 den ersten und zweiten Hebel 320, 330 in eine Ruheposition P zu überführen. Die Rückstellfeder 332 weist hierbei eine flache Auflage 333 auf, gegen die die Rückstellfeder 332 wirkt, sodass sich eine Rückstellkraft ergibt, welche den zweiten Hebel nach oben - also in Richtung des ersten Hebels 320 - drückt, wodurch der obere Hebel 320 wiederum zurückschnappt und in Richtung der Ruheposition P gedrückt wird, sofern der erste Hebel 320 nicht betätigt wird.

Die Aufhängung 326 des ersten Hebels 320 und die Aufhängung 336 des zweiten Hebels 330, beziehungsweise deren Schwenkachsen X, Y, sind parallel zueinander angeordnet und stehen überdies senkrecht auf einer Ebene, welche entlang der Drehachse M verläuft. Hierdurch wird eine besonders vorteilhafte Kraftübertragung vom Hebelmechanismus 304 auf das auf der Drehachse M positionierte Löseelement 210 bewerkstelligt.

Weiterhin ist die Aufhängung 326 des ersten Hebels 320 und die Aufhängung 336 des zweiten Hebels 330, respektive deren Schwenkachsen X, Y, in Bezug auf die Drehachse M einander gegenüberliegend an dem Deckel angeordnet, wobei durch die zweiarmige Ausführung des ersten Hebels 320 und die einarmige Ausführung des zweiten Hebels 330 eine besonders effiziente Kraftübertragung ermöglicht ist. Durch die übereinanderliegende Anordnung des ersten und zweiten Hebels 320, 330 sind weiterhin auch in Richtung der Drehachse M die beiden Schwenkachsen X, Y zueinander leicht versetzt.

In der Ruheposition P ist der zweite Hebel 330 von dem Löseelement 210 beabstandet, sodass eine unbeabsichtigte Kontaktierung dieser beiden Elemente ausgeschlossen ist.

Weiterhin weist der erste Hebel 320 bevorzugt an der Unterseite des Griffelements 322 ein Rastelement 325 auf, welches in der Ruheposition P mit dem zweiten Hebel 330 interagiert und verrastet. Hierdurch erhält der Benutzer ein haptisches wie auch akustisches Feedback, sofern sich die beiden Hebel 320, 330 korrekt in der Ruheposition P befinden. Dies erhöht auch die Betriebssicherheit des Handinstruments 1, da durch die Verrastung eine zusätzliche mechanische Sicherung des Hebelmechanismus 304 gebildet ist, sodass ein unbeabsichtigtes Anheben des ersten Hebels 320 und somit ein Betätigen B des Hebelmechanismus 304 verhindert wird.

Durch die zweiteilige Ausführung des Hebelmechanismus 304 wird die durch den Benutzer aufzubringende Kraft erheblich verringert. Mit einem einteiligen Hebelmechanismus, wäre zwar auch eine Erleichterung spürbar, allerdings würde dann bei Betätigung der eine Hebel zwangsläufig mit dem Löseelement 210 reiben, da sich die Kontaktfläche des einen Hebels dann auf dem Löseelement 210 seitlich verschiebt, wobei dadurch ein nachteiliger Materialabtrag an beiden Elementen resultiert. Dies würde die Betriebssicherheit des Handinstruments 1 erheblich verringern. Insofern erweist sich der zweiteilige Hebelmechanismus 304 für die vorliegende Anwendung als vorteilhaft.

In den Figuren 3A, 3B, 4A und 4B ist eine beispielhafte zweite Ausführungsform eines erfindungsgemäßen Deckels 30 gezeigt, welcher mit einem Handinstrument 1 reversibel koppelbar ist. Im Wesentlichen ist der Aufbau dieser zweiten Ausführungsform identisch mit der bereits aus Figur 2 bekannten beispielhaften ersten Ausführungsform, wobei durch die Darstellung ohne die übrigen Komponenten des Handinstruments 1 und somit insbesondere auch ohne die Darstellung der Komponenten des Haltemechanismus, der räumliche Aufbau des Deckels 30 klarer ersichtlich ist. Die Figuren 3B und 4B geben hierfür eine perspektivische Ansicht auf den Deckel 30 und dessen Hebelmechanismus 304, der identisch zur ersten Ausführungsform ist.

Die zweite Ausführungsform bringt ein weiteres zusätzliches Feature mit sich, welches die Bedienbarkeit und die Bedienungssicherheit zusätzlich erhöht. Hierbei ist eine zusätzliche Feststellvorrichtung 310 vorgesehen, welche es ermöglicht eine eingestellte Orientierung bezüglich der Drehachse M des Hebelmechanismus 304 zu arretieren, derart, dass nach Arretierung ein unbeabsichtigtes Verdrehen des Mittelstücks 301 verhindert ist. Selbstverständlich kann die Arretierung der Feststellvorrichtung 310 wieder gelöst werden, sodass der Hebelmechanismus 304 und damit verbunden auch das Mittelstück 301 umorientiert werden kann, um dann wieder arretiert zu werden. Wie aus den Figuren 3A bis 4B ersichtlich ist die Feststellvorrichtung 310 in der hier dargestellten Variante eine Übermuttervorrichtung, bestehend aus einem Ringelement 311, welches wiederum ein Gewinde hat, und dazu ausgebildet ist, mit einem auf der Innenseite des Gewindestücks angeordneten Innengewinde 303 zu interagieren. Das Ringelement 311 ist hierbei zwischen Mittelstück 301 und Außenstück 305 angeordnet, und fixiert, sofern das Ringelement 311 entsprechend eingeschraubt ist, das Mittelstück 301 durch Kraftschluss. Das Ringelement 311 weist hierfür zum Einschrauben entsprechende Werkzeugausbuchtungen 319 auf, sodass eine feste Arretierung ermöglicht ist. Durch diese Ausführung mit der Feststellvorrichtung 310 kann das Handinstrument 1 beziehungsweise der Deckel 30 an die individuellen Anforderungen und Präferenzen des Benutzers angepasst werden, sodass beispielsweise ein Linkshänder den Hebelmechanismus 304 anders orientieren kann, wodurch die Ergonomie des Handinstruments 1 wesentlich erhöht wird.

Weiterhin kann auch das Außenstück 305 entsprechende Werkzeugausbuchtungen 309 aufweisen, sodass ein festes verschrauben des Deckels 30 mit dem Kopfstück 20 des Handinstruments 1 ermöglicht ist, insbesondere derart, dass ein werkzeugloses Entkoppeln dieser Komponenten nicht möglich ist, was die Betriebssicherheit des Handinstruments 1 weiter erhöht.

Wie weiterhin aus den Figuren 3A und 4B ersichtlich ist, kann sich das Außenstück 305 auch unterhalb des Mittelstücks 301 erstrecken, wobei hierdurch die Stabilität des Deckels erhöht wird. Hierbei kann weiterhin vorgesehen sein, dass das Mittelstück 301 über ein Kugellager 312 im Außenstück 305 drehbar gelagert ist, sodass ein Umorientieren des Mittelstücks 301 und somit auch des Hebelmechanismus 304 entlang der Drehachse M vereinfacht ist. Mittels der Feststellvorrichtung 310 wird dann das Kugellager 312 ebenfalls arretiert, sodass ein weiteres verdrehen des Mittelstücks 301 verhindert ist. Diese Ausgestaltungen sind als Ganzes oder auch einzeln ebenfalls auf die zuvor in Figur 2 gezeigte erste Ausführungsform anwendbar.

Der Deckel 30 weist weiterhin auch einen Aufnahmeraum 350 auf, in welchem Komponenten des Kopfstücks 20 aufgenommen werden, wenn der Deckel 30 mit dem Handinstrument 1 gekoppelt ist. Wichtig ist hierbei, dass die Bewegungsfreiheit des zweiten Hebels 330 nicht eingeschränkt wird, sodass die Durchbiegung 331 bei Betätigung B des Hebelmechanismus 304 mit dem Löseelement 210 des Kopfteils 20 interagieren kann. Dies ist auch auf die zuvor in Figur 2 gezeigten Ausführung übertragbar.

In den Figuren 3A und 3B sind weiterhin Betätigungspositionen B des Hebelmechanismus 304 gezeigt, wobei in Zusammenschau mit den Figuren 4A und 4B, welche die Ruheposition P des Hebelmechanismus 304 zeigen, die Möglichen Hebelstellungen verdeutlicht sind. Diese Darstellung der Betätigung B und der Ruheposition sind hierbei auf sämtliche beispielhafte Ausführungsformen übertragbar, wobei selbstverständlich auch anders angeordnete zweiteilig ausgeführte Hebelmechanismen denkbar sind.

Wie insbesondere aus den Figuren 4A und 4B ersichtlich ist der Hebelmechanismus 304 so gebildet, dass der erste Hebel 320 sich in die Oberfläche des Mittelstücks 301 plan integriert. Lediglich das Griffelement 322 steht etwas über dem Mittelstück 301 hervor, sodass dieses besonders einfach durch einen Benutzer bedient werden kann.

In Figur 5 ist eine weitere beispielhafte dritte Ausführungsform des Deckels 30 beziehungsweise Handinstruments 1 dargestellt. Neben dem aus der ersten und zweiten Ausführungsform bekannten zweiteiligen Hebelmechanismus 304 weist diese Ausführungsform einen weiteren Mechanismus zur Lösung des Haltemechanismus auf.

Hierbei ist vorgesehen, dass zwischen Deckel 30 und Kopfstück 20 ein Federelement 40 angeordnet ist. Der Deckel 30 und somit auch der Hebelmechanismus 304 ist in dieser Ausführung entlang der Drehachse M beweglich gelagert, wobei das Federelement 40 den Deckel 30 vom Kopfstück 20 wegdrückt. Insofern ist der Hebelmechanismus 304 mittels der Federkraft des Federelements 40 in eine von dem Löseelement 210 beabstandete Position vorgespannt.

Die Kopplung des Deckels 30 mit dem Kopfstück 20 erfolgt hierbei ebenfalls über das Gewinde 302 und das Gegengewinde 220, allerdings ist diese Kopplung nun derart ausgeführt, dass nach Aufschrauben des Deckels 30 über die Gewinde 302, 220 der Deckel 30 entlang einer glatten Oberfläche hin und her verschoben werden kann, wobei die Gewinde 302, 220 ein unbeabsichtigtes Entkoppeln des Deckels 30 vom Kopfstück 20 verhindern. Der Hebelmechanismus 304 ist also in dieser Ausführungsform entgegen der Federkraft herabdrückbar, um in einer Ruheposition der beiden Hebel 320, 330 im herabgedrückten Zustand das Löseelement 210 zu kontaktieren.

Insofern ist mit dieser beispielhaften dritten Ausführungsform sowohl der Hebelmechanismus 304 als auch ein Druckknopfmechanismus zur Lösung des Haltemechanismus im Deckel 30 integriert, sodass ein Benutzer die Wahl hat, welchen Mechanismus er verwendet. In dieser Ausführung kann beispielsweise vorgesehen sein, ein besonders starkes Federelement 40 zu verwenden, welches durch leichte Berührungen beziehungsweise Krafteinwirkungen auf den Deckel 30 nicht zusammengestaucht wird, sodass ein unbeabsichtigtes Kontaktieren des Hebelmechanismus 304 während des Betriebs des Handinstruments 1 verhindert wird. Hierbei wäre allerdings ebenfalls eine große Kraftanstrengung durch den Benutzer des Handinstruments 1 von Nöten, um das Behandlungswerkzeug 7 mit dem Handinstrument 1 zu koppeln (also zu spannen) oder zu entkoppeln (also zu lösen). Benutzer, welche diese erforderliche Kraft nicht aufbringen können, können dann den Hebelmechanismus 304 verwenden, um in einfacher Weise den Haltemechanismus des Handinstruments 1 zu lösen.

Sämtliche der zuvor beschriebenen beispielhaften Ausführungen des Handinstruments 1 und des Deckels 30 erleichtern das Lösen des Behandlungswerkzeugs 7 in erheblicher Weise. Der Deckel 30 ist hierbei vorteilhaft so ausgestaltet, dass er ebenfalls auf ältere bereits bekannte Handinstrumente 1 aufschraubbar ist, sodass der erfindungsgemäße zweiteilige Hebelmechanismus 304 auch auf älteren Handinstrumente 1 angewendet werden kann. Mit der Ausgestaltung eines abnehmbaren Deckels 30 lassen sich besonders einfach und vorteilhaft Wartungs-, Reinigungs- bzw. Reparaturarbeiten im Inneren des Kopfstücks 20 wie auch am Deckel 30 selbst durchführen. Was eine besonders lange Lebensdauer des Handinstruments 1 und des Deckels 30 ermöglicht.

Das so beschaffene Handinstrument 1, respektive der so beschaffene Deckel 30 für ein Handinstrument 1, ermöglicht somit eine besonders einfache und sichere Bedienung zur Lösung des Haltemechanismus eines Handinstruments 1 für ein Behandlungswerkzeug 7. Unbeabsichtigte Betätigungen des Mechanismus werden so vermieden, wobei zudem auch eine Kontaktierung des Hebelmechanismus 304 mit dem Löseelement 210 insbesondere im Betrieb verhindert wird, sodass keine Verbrennungsgefahr für den Patienten oder den behandelnden Arzt besteht.

## Patentansprüche

1. Medizinisches, insbesondere dentalmedizinisches Handinstrument (1) mit einer Griffhülse (10) und einem Kopfstück (20), welches an einem vorderen Ende (101) der Griffhülse (10) angeordnet ist und eine Werkzeugaufnahmeöffnung (240) aufweist, wobei im Inneren des Kopfstücks (20) ein Haltemittel (230) zur drehbaren Halterung eines Behandlungswerkzeugs (7), beispielsweise eines zahnärztlichen Bohrers, angeordnet ist,
wobei das Haltemittel (230) entlang einer Drehachse (M) für das Behandlungswerkzeug (7) angeordnet ist, und
wobei im Inneren des Kopfstücks (20) entlang der Drehachse (M) ein Löseelement (210) angeordnet ist, welches dazu ausgebildet ist, mit dem Haltemittel (230) zu interagieren, wobei das Löseelement (210) bei einer Interaktion mit dem Haltemittel (230) die Halterung des Behandlungswerkzeugs (7) löst,
wobei das Kopfstück (20) weiterhin einen Deckel (30) aufweist, wobei der Deckel (30) einen Hebelmechanismus (304) umfasst, der einen ersten Hebel (320) und einen zweiten Hebel (330) aufweist, welche zusammenwirken um die Halterung des Behandlungswerkzeugs (7) zu lösen,
**dadurch gekennzeichnet,**
**dass** der erste Hebel (320) bei Betätigung auf ein freies Ende des zweiten Hebels (330) wirkt.

2. Medizinisches Handinstrument gemäß Anspruch 1,
**dadurch gekennzeichnet,**
**dass** eine Schwenkachse (X) des ersten Hebels (320) und eine Schwenkachse (Y) des zweiten Hebels (330) parallel zueinander angeordnet sind und senkrecht auf einer Ebene stehen, welche entlang der Drehachse (M) verläuft.

3. Medizinisches Handinstrument nach Anspruch 2,
**dadurch gekennzeichnet,**
**dass** die Schwenkachse (X) des ersten Hebels (320) und die Schwenkachse (Y) des zweiten Hebels (330) in Bezug auf die Drehachse (M) einander gegenüberliegend an dem Deckel angeordnet sind,
wobei in Richtung der Drehachse (M) beide Schwenkachsen (X, Y) vorzugsweise zueinander versetzt sind.

4. Medizinisches Handinstrument gemäß einem der vorherigen Ansprüche,
**dadurch gekennzeichnet,**
**dass** der zweite Hebel (330) eine Durchbiegung (331) aufweist, welche zur Interaktion mit dem Löseelement (210) auf dieses drückt, wobei durch die Durchbiegung (331) eine auf das Löseelement (210) wirkende Kraft begrenzt wird.

5. Medizinisches Handinstrument gemäß einem der vorherigen Ansprüche,
**dadurch gekennzeichnet,**
**dass** der zweite Hebel (330) im Bereich seiner Schwenkachse (Y) mit einer Rückstellfeder (332), insbesondere einer Schenkelfeder, versehen ist, welche dazu ausgebildet ist, bei ausbleibender Betätigung (B) des ersten Hebels (320) den ersten und zweiten Hebel (320, 330) in eine Ruheposition (P) zu überführen,
wobei in der Ruheposition (P) der zweite Hebel (330) von dem Löseelement (210) beabstandet ist.

6. Medizinisches Handinstrument gemäß einem der vorherigen Ansprüche,
**dadurch gekennzeichnet,**
**dass** der erste Hebel (320) an seinem der Schwenkachse (X) zugewandten Endbereich mit seitlichen Vorwölbungen (323) versehen ist, welche der Lagerung des Hebels (320) in dem Deckel (30) dienen und den Endbereich des zweiten Hebels (330) jeweils seitlich übergreifen.

7. Medizinisches Handinstrument gemäß einem der vorherigen Ansprüche,
**dadurch gekennzeichnet,**
**dass** der erste Hebel (320) an seinem von der Schwenkachse (X) entfernten Endbereich mit einem Griffelement (322) versehen ist.

8. Medizinisches Handinstrument gemäß einem der vorherigen Ansprüche,
**dadurch gekennzeichnet,**
**dass** der erste Hebel (320) an seiner dem zweiten Hebel (330) zugewandten Unterseite mit einer Ausnehmung (324) versehen ist, welche in einer Ruheposition (P) der beiden Hebel (320, 330) zumindest einen Teil des zweiten Hebels (330) aufnimmt.

9. Medizinisches Handinstrument gemäß einem der vorherigen Ansprüche,
**dadurch gekennzeichnet,**
**dass** der Deckel (30) reversibel mit dem Kopfstück (20) des Handinstruments (1) koppelbar ist,
wobei hierfür bevorzugt der Deckel (30) ein Gewinde (302) und das Kopfstück (20) ein entsprechendes Gegengewinde (220) aufweist.

10. Medizinisches Handinstrument gemäß einem der vorherigen Ansprüche,
**dadurch gekennzeichnet,**
**dass** der Deckel (30) eine Feststellvorrichtung (310) aufweist, wobei der Hebelmechanismus (304) mittels der Feststellvorrichtung (310) wahlweise in einer gewünschten Orientierung bezüglich der Drehachse (M) arretierbar ist.

11. Medizinisches Handinstrument gemäß einem der vorherigen Ansprüche 1 bis 9,
**dadurch gekennzeichnet,**
**dass** der Hebelmechanismus (304) entlang der Drehachse (M) beweglich gelagert ist und
**dass** der Hebelmechanismus (304) mittels Federkraft in eine von dem Löseelement (210) beabstandete Position vorgespannt ist,
wobei der Hebelmechanismus (304) entgegen der Federkraft herabdrückbar ist, um in einer Ruheposition der beiden Hebel (320, 330) im herabgedrückten Zustand das Löseelement (210) zu kontaktieren.

12. Medizinisches Handinstrument gemäß einem der vorherigen Ansprüche 1 bis 11,
**dadurch gekennzeichnet,**
**dass** das Haltemittel (230) eine Spannzange ist, und/oder das Löseelement (210) ein Keilelement mit einem keilförmigen Kopfbereich (211) und einem planen Bodenbereich (212) ist.

13. Deckel (30) für ein Kopfstück (20) eines medizinischen, insbesondere dentalmedizinischen Handinstruments (1) mit einer Griffhülse (10) und einem Kopfstück (20), welches an einem vorderen Ende (101) der Griffhülse (10) angeordnet ist und eine Werkzeugaufnahmeöffnung (240) aufweist,
wobei im Inneren des Kopfstücks (20) ein Haltemittel (230) zur drehbaren Halterung eines Behandlungswerkzeugs (7), beispielsweise eines zahnärztlichen Bohrers, angeordnet ist,
wobei das Haltemittel (230) entlang einer Drehachse (M) für das Behandlungswerkzeug (7) angeordnet ist, und
wobei im Inneren des Kopfstücks (20) entlang der Drehachse (M) ein Löseelement (210) angeordnet ist, welches dazu ausgebildet ist mit dem Haltemittel (230) zu interagieren, wobei das Löseelement (210) bei einer Interaktion mit dem Haltemittel (230) die Halterung des Behandlungswerkzeugs (7) löst,
wobei der Deckel (30) einen Hebelmechanismus (304) umfasst, der einen ersten Hebel (320) und einen zweiten Hebel (330) aufweist, welche zusammenwirken, um die Halterung des Behandlungswerkzeugs (7) zu lösen,
**dadurch gekennzeichnet,**
**dass** der erste Hebel (320) bei Betätigung auf ein freies Ende des zweiten Hebels (330) wirkt.

14. Deckel für ein Kopfstück gemäß Anspruch 13,
**dadurch gekennzeichnet,**
**dass** der erste Hebel (320) als zweiarmiger Hebel und der zweite Hebel (320) als einarmiger Hebel ausgebildet ist.

## Claims

1. Medical, in particular dental hand-held instrument (1) having a grip sleeve (10) and a headpiece (20) which is arranged at a front end (101) of the grip sleeve (10) and has a tool-receiving opening (240), wherein a holding means (230) for rotatably securing a treatment tool (7), for example a dental drill, is arranged in the interior of the headpiece (20),
wherein the holding means (230) is arranged along an axis of rotation (M) for the treatment tool (7), and
wherein a release element (210) is arranged in the interior of the headpiece (20) along the axis of rotation (M), said release element being designed to interact with the holding means (230), wherein the release element (210) releases the securing of the treatment tool (7) upon interaction with the holding means (230),
wherein the headpiece (20) furthermore has a cover (30), wherein the cover (30) comprises a lever mechanism (304) which has a first lever (320) and a second lever (330) which cooperate in order to release the securing of the treatment tool (7),
**characterized**
**in that** the first lever (320), upon actuation, acts on a free end of the second lever (330).

2. Medical hand-held instrument according to Claim 1,
**characterized**
**in that** a pivot axis (X) of the first lever (320) and a pivot axis (Y) of the second lever (330) are arranged parallel to each other and are perpendicular to a plane which extends along the axis of rotation (M).

3. Medical hand-held instrument according to Claim 2,
**characterized**
**in that** the pivot axis (X) of the first lever (320) and the pivot axis (Y) of the second lever (330) are arranged on the cover opposite each other in relation to the axis of rotation (M),
wherein, in the direction of the axis of rotation (M), the two pivot axes (X, Y) are preferably offset from each other.

4. Medical hand-held instrument according to any one of the preceding claims,
**characterized**
**in that** the second lever (330) has a bend (331) which, to interact with the release element (210), presses on the latter, wherein a force acting on the release element (210) is limited by the bend (331).

5. Medical hand-held instrument according to any one of the preceding claims,
**characterized**
**in that** the second lever (330) is provided, in the region of its pivot axis (Y), with a return spring (332), in particular a leg spring, which is designed, in the absence of actuation (B) of the first lever (320), to transfer the first and second levers (320, 330) into a rest position (P),
wherein, in the rest position (P), the second lever (330) is spaced apart from the release element (210).

6. Medical hand-held instrument according to any one of the preceding claims,
**characterized**
**in that** the first lever (320) is provided, at its end region facing the pivot axis (X), with lateral protrusions (323) which serve for mounting the lever (320) in the cover (30) and each engage laterally over the end region of the second lever (330).

7. Medical hand-held instrument according to any one of the preceding claims,
**characterized**
**in that** the first lever (320) is provided, at its end region remote from the pivot axis (X), with a grip element (322).

8. Medical hand-held instrument according to any one of the preceding claims,
**characterized**
**in that** the first lever (320) is provided, on its underside facing the second lever (330), with a recess (324) which, in a rest position (P) of the two levers (320, 330), receives at least a part of the second lever (330).

9. Medical hand-held instrument according to any one of the preceding claims,
**characterized**
**in that** the cover (30) is able to be coupled reversibly to the headpiece (20) of the hand-held instrument (1), wherein preferably, for this purpose, the cover (30) has a thread (302) and the headpiece (20) has a corresponding mating thread (220).

10. Medical hand-held instrument according to any one of the preceding claims,
**characterized**
**in that** the cover (30) has a fixing device (310), wherein the lever mechanism (304) is able to be locked selectively in a desired orientation with respect to the axis of rotation (M) by means of the fixing device (310).

11. Medical hand-held instrument according to any one of the preceding Claims 1 to 9,
**characterized**
**in that** the lever mechanism (304) is mounted movably along the axis of rotation (M), and
**in that** the lever mechanism (304) is preloaded by means of spring force into a position spaced apart from the release element (210),
wherein the lever mechanism (304) is able to be depressed counter to the spring force in order, in the depressed state, to contact the release element (210) in a rest position of the two levers (320, 330).

12. Medical hand-held instrument according to any one of the preceding Claims 1 to 11,
**characterized**
**in that** the holding means (230) is a collet, and/or the release element (210) is a wedge element having a wedge-shaped head region (211) and a planar bottom region (212).

13. Cover (30) for a headpiece (20) of a medical, in particular dental hand-held instrument (1) having a grip sleeve (10) and a headpiece (20) which is arranged at a front end (101) of the grip sleeve (10) and has a tool-receiving opening (240),
wherein a holding means (230) for rotatably securing a treatment tool (7), for example a dental drill, is arranged in the interior of the headpiece (20),
wherein the holding means (230) is arranged along an axis of rotation (M) for the treatment tool (7), and
wherein a release element (210) is arranged in the interior of the headpiece (20) along the axis of rotation (M), said release element being designed to interact with the holding means (230), wherein the release element (210) releases the securing of the treatment tool (7) upon interaction with the holding means (230),
wherein the cover (30) comprises a lever mechanism (304) which has a first lever (320) and a second lever (330) which cooperate in order to release the securing of the treatment tool (7),
**characterized**
**in that** the first lever (320), upon actuation, acts on a free end of the second lever (330).

14. Cover for a headpiece according to Claim 13,
**characterized**
**in that** the first lever (320) is in the form of a two-armed lever and the second lever (320) is in the form of a one-armed lever.

## Revendications

1. Instrument médical portatif, en particulier un instrument dentaire (1), comportant un manchon de préhension (10) et une tête (20) disposée à une extrémité avant (101) du manchon de préhension (10) et présentant une ouverture de réception d'outil (240), un moyen de maintien (230) étant disposé à l'intérieur de la tête (20) pour le maintien rotatif d'un outil de traitement (7), par exemple d'une fraise dentaire,
le moyen de maintien (230) étant disposé le long d'un axe de rotation (M) pour l'outil de traitement (7), et
un élément de libération (210) étant disposé à l'intérieur de la tête (20) le long de l'axe de rotation (M), cet élément étant conçu pour interagir avec le moyen de maintien (230), l'élément de libération (210) libérant le maintien de l'outil de traitement (7) lors d'une interaction avec le moyen de maintien (230),
la tête (20) comportant en outre un couvercle (30), le couvercle (30) comprenant un mécanisme à levier (304) qui comporte un premier levier (320) et un second levier (330) qui coopèrent pour libérer le maintien de l'outil de traitement (7),
**caractérisé en ce que**
le premier levier (320) agit, lors de son actionnement, sur une extrémité libre du second levier (330).

2. Instrument médical portatif selon la revendication 1,
**caractérisé en ce que**
un axe de pivotement (X) du premier levier (320) et un axe de pivotement (Y) du second levier (330) sont disposés parallèlement l'un à l'autre et perpendiculairement à un plan s'étendant le long de l'axe de rotation (M).

3. Instrument médical portatif selon la revendication 2,
**caractérisé en ce que**
l'axe de pivotement (X) du premier levier (320) et l'axe de pivotement (Y) du second levier (330) sont disposés de manière opposée l'un à l'autre par rapport à l'axe de rotation (M) sur le couvercle,
les deux axes de pivotement (X, Y) étant de préférence décalés l'un par rapport à l'autre dans la direction de l'axe de rotation (M).

4. Instrument médical portatif selon l'une quelconque des revendications précédentes,
**caractérisé en ce que**
le second levier (330) présente une déformation (331) qui, pour l'interaction avec l'élément de libération (210), exerce une pression sur celui-ci, une force agissant sur l'élément de libération (210) étant limitée par la déformation (331).

5. Instrument médical portatif selon l'une quelconque des revendications précédentes,
**caractérisé en ce que**
le second levier (330) est pourvu, dans la zone de son axe de pivotement (Y), d'un ressort de rappel (332), notamment d'un ressort à branches, conçu pour ramener les premier et second leviers (320, 330) dans une position de repos (P) en l'absence d'actionnement (B) du premier levier (320),
le second levier (330) étant espacé de l'élément de libération (210) dans la position de repos (P).

6. Instrument médical portatif selon l'une quelconque des revendications précédentes,
**caractérisé en ce que**
le premier levier (320) est pourvu, à son extrémité tournée vers l'axe de pivotement (X), de protubérances latérales (323) qui servent au support du levier (320) dans le couvercle (30) et qui s'engagent respectivement latéralement sur la zone d'extrémité du second levier (330).

7. Instrument médical portatif selon l'une quelconque des revendications précédentes,
**caractérisé en ce que**
le premier levier (320) est pourvu, à son extrémité éloignée de l'axe de pivotement (X), d'un élément de préhension (322).

8. Instrument médical portatif selon l'une quelconque des revendications précédentes,
**caractérisé en ce que**
le premier levier (320) est pourvu, sur sa face inférieure tournée vers le second levier (330), d'un évidement (324) qui, dans une position de repos (P) des deux leviers (320, 330), reçoit au moins une partie du second levier (330).

9. Instrument médical portatif selon l'une quelconque des revendications précédentes,
**caractérisé en ce que**
le couvercle (30) peut être accouplé de manière réversible à la tête (20) de l'instrument portatif (1), le couvercle (30) présentant à cet effet de préférence un filetage (302) et la tête (20), un filetage complémentaire (220).

10. Instrument médical portatif selon l'une quelconque des revendications précédentes,
**caractérisé en ce que**
le couvercle (30) comporte un dispositif de blocage (310), le mécanisme à levier (304) pouvant être bloqué au moyen du dispositif de blocage (310) dans une orientation souhaitée par rapport à l'axe de rotation (M) .

11. Instrument médical portatif selon l'une quelconque des revendications 1 à 9 précédentes,
**caractérisé en ce que**
le mécanisme à levier (304) est monté mobile le long de l'axe de rotation (M) et
**en ce que** le mécanisme à levier (304) est précontraint par une force de ressort dans une position espacée de l'élément de libération (210),
le mécanisme à levier (304) pouvant être enfoncé à l'encontre de la force de ressort pour, dans une position de repos des deux leviers (320, 330), entrer en contact avec l'élément de libération (210) dans l'état enfoncé.

12. Instrument médical portatif selon l'une quelconque des revendications 1 à 11 précédentes,
**caractérisé en ce que**
le moyen de maintien (230) est une pince de serrage, et/ou l'élément de libération (210) est un élément en coin ayant une zone de tête en forme de coin (211) et une zone de fond plane (212).

13. Couvercle (30) pour une tête (20) d'un instrument médical portatif, en particulier d'un instrument dentaire (1), comprenant un manchon de préhension (10) et une tête (20) disposée à une extrémité avant (101) du manchon de préhension (10) et présentant une ouverture de réception d'outil (240),
un moyen de maintien (230) étant disposé à l'intérieur de la tête (20) pour le maintien rotatif d'un outil de traitement (7), par exemple d'une fraise dentaire,
le moyen de maintien (230) étant disposé le long d'un axe de rotation (M) pour l'outil de traitement (7), et
un élément de libération (210) étant disposé à l'intérieur de la tête (20) le long de l'axe de rotation (M), cet élément étant conçu pour l'interaction avec le moyen de maintien (230), l'élément de libération (210) libérant le maintien de l'outil de traitement (7) lors d'une interaction avec le moyen de maintien (230),
le couvercle (30) comprenant un mécanisme à levier (304) qui comporte un premier levier (320) et un second levier (330) qui coopèrent pour libérer le maintien de l'outil de traitement (7),
**caractérisé en ce que**
le premier levier (320) agit, lors de son actionnement, sur une extrémité libre du second levier (330).

14. Couvercle pour une tête selon la revendication 13,
**caractérisé en ce que**
le premier levier (320) est conçu sous la forme d'un levier à deux bras et le second levier (320), sous la forme d'un levier à un bras.
